# FASCICULE DE BREVET EUROPEEN

(11) **EP 2 190 376 B1**
(45) Date de publication et mention de la délivrance du brevet: **24.05.2017**
(21) Numéro de dépôt: 08803100.0
(22) Date de dépôt: 20.08.2008
(51) Int. Cl.: A61B 90/00

(54) **SYSTÈME D'IMAGERIE POUR LE SUIVI D'UN OUTIL CHIRURGICAL DANS UN CHAMP OPÉRATOIRE**
BILDGEBUNGSSYSTEM ZUR VERFOLGUNG EINES OPERATIONSWERKZEUGS IN EINEM OPERATIONSFELD
IMAGING SYSTEM FOR FOLLOWING A SURGICAL TOOL IN AN OPERATION FIELD

(30) Priorité: 24.08.2007 FR 0757158
(43) Date de publication de la demande: 02.06.2010
(73) Titulaire: Endocontrol, 38700 La Tronche (FR); Université Grenoble Alpes, 38400 Saint-Martin-d'Hères (FR)
(72) Inventeur: CINQUIN, Philippe, F-38330 St Nazaire Les Eymes (FR); MOZER, Pierre, F-94300 Vincennes (FR); VOROS, Sandrine, F-92310 Sevres (FR); LONG, Jean-Alexandre, F-38190 Bernin (FR); DUCHATEAU, Josselin, F-38000 Grenoble (FR); MOREAU-GAUDRY, Alexandre, F-38000 Grenoble (FR); VIDAL, Clément, F-38000 Grenoble (FR); HENRI, Patrick, F-92600 Asnieres (FR)
(74) Mandataire: Regimbeau
(86) Numéro de dépôt international: PCT/EP2008/060867
(87) Numéro de publication internationale: WO 2009/027277

(56) Documents cités:
- EP-A- 1 561 420
- WO-A-99/58065
- WO-A-2004/042546
- WO-A-2006/008300
- US-A1- 2001 016 684
- US-A1- 2006 262 118

## Description

### DOMAINE DE L'INVENTION

La présente invention concerne le domaine de l'imagerie médicale, plus particulièrement pour le suivi d'un outil chirurgical dans un champ opératoire.

### ETAT DE LA TECHNIQUE

La chirurgie endoscopique nécessite un système d'imagerie performant pour la visualisation du champ opératoire de manière à aider le chirurgien dans son opération.

Un certain nombre de solutions ont été développées pour améliorer la visualisation offerte par l'image d'une caméra endoscopique classique.

Certains systèmes utilisent des images du champ opératoire acquises avant l'opération par un système quelconque du type scanner X, Imagerie par Résonnance Magnétique ou caméra stéréoscopique. Les images préopératoires permettent en effet de segmenter le volume 3D acquis avant l'opération pour isoler les organes d'intérêt, et les mettre en correspondance avec le système d'imagerie per-opératoire, consistant en général en une caméra endoscopique. La fusion des images 3D acquises avant l'opération avec les images endoscopiques acquises pendant l'opération offre au chirurgien une visualisation améliorée du champ opératoire puisque les organes d'intérêt sont mieux mis en évidence. Toutefois, les solutions existantes sont relativement lourdes à mettre en oeuvre. En outre, les images utilisant des informations préopératoires ne sont pas totalement fiables puisqu'il peut y avoir des modifications morphologiques entre l'acquisition des données et l'intervention (que ce soit des modifications de la position des organes entre eux ou même des modifications de leur forme).

Il a donc été développé des solutions basées uniquement sur l'exploitation de données acquises pendant l'opération pour s'affranchir de ces problèmes.

Il a par exemple été proposé d'utiliser, en plus des images endoscopiques, des images échographiques de manières à pouvoir visualiser des organes non visibles avec la caméra endoscopique, parce qu'ils sont cachés. Par exemple, la publication intitulée « Real-Time Transrectal Ultrasound Guidance During Laparoscopic Radical Prostatectomy : Impact on Surgical Margins » (Journal of Urology, Vol. 175, 1304-1310, April 2006) propose d'utiliser une sonde échographique endorectale manipulée par un opérateur humain pendant une prostatectomie radicale. Ceci permet d'améliorer de façon significative les résultats carcinologiques de l'intervention en permettant à l'opérateur de visualiser avec précision les limites de l'organe même si celles-ci sont situées en arrière des organes visibles dans le champ de la caméra endoscopique. Le praticien dispose ainsi d'une image échographique du champ opératoire pour voir très nettement les organes et leur positionnement relatif, et d'une image endoscopique pour voir l'outil chirurgical dans le champ opératoire. Document WO-A-2006/008300 montre un système comportant les caractéristiques défini dans le préambule de la revendication 1. Pour faciliter la démarche du praticien, il a été proposé de modifier les outils chirurgicaux pour qu'ils puissent être visibles non seulement dans l'image endoscopique mais également dans l'image échographique. Il a par exemple été proposé de placer sur l'outil chirurgical des transducteurs à ultrasons pour pouvoir détecter l'outil par le dispositif d'imagerie échographique, et permettre éventuellement un recalage entre l'image endoscopique et l'image échographique pour fusionner les informations endoscopiques et échographique en vue d'obtenir une image enrichie. Toutefois une telle solution est coûteuse en temps puisque le traitement d'informations est complexe, de sorte que la visualisation des images enrichies ne peut se faire en temps réel.

Une autre solution permettant de mettre en correspondance des images endoscopiques et des images échographiques est d'utiliser un localisateur optique, placé à l'extérieur du patient de manière à pouvoir visualiser à la fois la caméra endoscopique et la sonde échographique, de manière à pouvoir opérer une triangulation pour recaler les images endoscopiques et échographiques acquise en se basant sur le positionnement relatif des parties externes de la caméra endoscopique et de la sonde échographique. Toutefois, une telle solution ne peut pas être mise en oeuvre dans n'importe quelle condition d'opération puisqu'il faut pouvoir visualiser les deux dispositifs d'imagerie. Un tel système est en outre relativement volumineux ce qui est un inconvénient majeur par rapport à l'encombrement dans la salle d'opération.

Un but de la présente invention est de proposer un système d'imagerie pour le suivi d'un outil chirurgical dans un champ opératoire à l'intérieur d'une cavité d'un corps d'un animal, en particulier chez un humain, permettant de résoudre au moins l'un des inconvénients précités.

Un but de la présente invention est en particulier de proposer un système permettant une visualisation des images enrichies proche du temps réel, voire en temps réel.

### EXPOSE DE L'INVENTION

A cette fin, on propose un système d'imagerie pour le suivi d'au moins un outil chirurgical dans un champ opératoire à l'intérieur d'un volume dans un corps d'un animal, en particulier chez un humain, selon la revendication 1. D'autres aspects préférés mais non limitatifs du système d'imagerie sont les suivants :
- le dispositif de traitement comprend des moyens pour fusionner les informations endoscopiques et les informations échographiques à partir de la mise en correspondance et pour obtenir une représentation du champ opératoire à partir des informations fusionnées ;
- le système comprend en outre un dispositif d'affichage de la représentation du champ opératoire à partir des informations fusionnées ;
- les marqueurs sont formés d'une substance biocompatible comprenant un colorant permettant au marqueur d'être reconnu par la caméra endoscopique ;
- les marqueurs sont formés d'une substance biocompatible comprenant des particules fluorophores émettant un signal permettant au marqueur d'être reconnu par la caméra endoscopique ;
- les marqueurs sont formés d'une substance biocompatible comprenant un produit de contraste reconnu par le dispositif d'imagerie par ultrasons ;
- la substance formant les marqueurs présente une cohésion suffisante pour empêcher ladite substance de se séparer, cette substance se présentant sous la forme d'un liquide ou d'une pâte ;
- les marqueurs comprennent une enveloppe formée dans un matériau biocompatible transparent aux ultrasons. ;
- les marqueurs comprennent une source d'énergie ;
- les marqueurs comprennent un dispositif d'émission d'un signal lumineux reconnu par la caméra endoscopique, ledit dispositif d'émission étant alimenté par la source d'énergie ;
- les marqueurs comprennent un dispositif d'émission d'un signal ultrasons reconnu par le dispositif d'imagerie par ultrasons, ledit dispositif d'émission étant alimenté par la source d'énergie ;
- le dispositif d'émission d'un signal ultrasons comprend au moins un transducteur piézo-électrique ;
- le dispositif d'émission d'un signal ultrasons comprend au moins un transducteur capacitif à ultrasons micro-usiné (CMUT) ;
- le dispositif de réception d'un signal ultrasons peut comprendre au moins un transducteur piezo-électrique ;
- le dispositif de réception d'un signal ultrasons peut comprendre au moins un transducteur capacitif à ultrasons micro-usiné (CMUT) ;
- les marqueurs présentent une cavité remplie d'un fluide comprenant un produit de contraste reconnu par le dispositif d'imagerie par ultrasons ;
- les marqueurs comprennent une portion recouverte d'un matériau rétro-réfléchissant biocompatible reconnu par la caméra endoscopique ;
- les marqueurs comprennent une portion recouverte d'une peinture biocompatible reconnue par la caméra endoscopique ;
- la peinture comprend des particules fluorophores, lesdites particules fluorophores émettant un signal reconnu par la caméra endoscopique ;
- les marqueurs comprennent des moyens de fixation pour être accrochés à l'intérieur du volume au niveau du champ opératoire ;
- les marqueurs sont fixes les uns par rapport aux autres ;
- les marqueurs sont reliés entre eux par une structure pour éviter le mouvement des marqueurs les uns par rapport aux autres ;
- le dispositif d'imagerie par ultrasons consiste en une sonde destinée à être introduite dans le corps de l'animal;
- le système comprend au moins un robot apte à porter au moins un moyen d'imagerie parmi la caméra endoscopique et le dispositif d'imagerie par ultrasons, le robot permettant un déplacement contrôlé dudit moyen d'imagerie.

### DESCRIPTION DES FIGURES

D'autres caractéristiques et avantages ressortiront encore de la description qui suit, laquelle est purement illustrative et non limitative et doit être lue en regard des figures annexées parmi lesquelles :
- La figure 1 est une représentation schématique du système d'imagerie pour le suivi d'un outil dans un champ opératoire située dans une cavité d'un patient ;
- La figure 2 est une représentation schématique d'un mode de réalisation d'un marqueur utilisé dans le système d'imagerie de la figure 1 ;
- La figure 3 est une représentation schématique d'un autre mode de réalisation d'un marqueur utilisé dans le système d'imagerie de la figure 1.

### DESCRIPTION DETAILLEE DE L'INVENTION

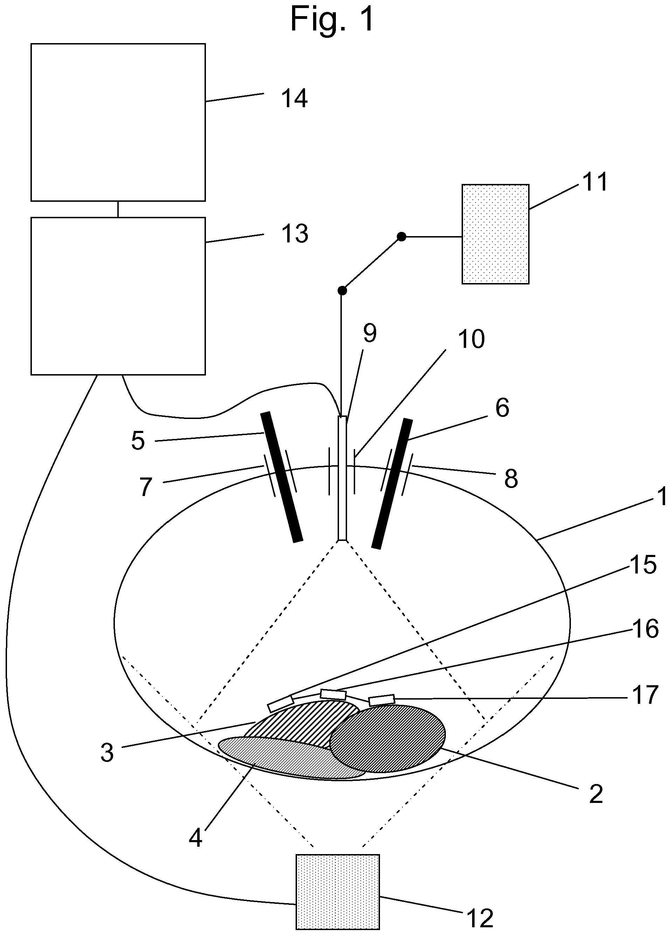

La figure 1 est une représentation schématique du système d'imagerie pour le suivi d'outils chirurgicaux dans le champ opératoire à l'intérieur d'un volume 1 dans le corps d'un patient, dans le cadre d'une intervention endoscopique. Ce volume 1, ou région volumique, forme une cavité 1, cette cavité étant naturelle ou artificielle créée dans ce cas par injection d'air dans le volume.

Comme cela est illustré schématiquement à la figure 1, il est pratiqué dans la cavité 1 une ou plusieurs incisions, qui serviront à l'insertion des outils chirurgicaux (5;6). Ces incisions sont réalisées à l'aide de trocarts (7;8) qui sont insérés à travers la paroi formant la cavité 1, et qui serviront de points d'entrée pour les outils à introduire à l'intérieur de la cavité 1.

Lors d'une opération sous endoscopie, il est critique que le chirurgien puisse visualiser correctement ses outils (5;6) par rapport aux organes (2;3;4) présents dans le champ opératoire.

A cette fin, le système d'imagerie comprend tout d'abord des moyens classiques d'imagerie photosensibles insérés dans la cavité 1 par voie endoscopique. On utilisera classiquement une ou plusieurs caméras endoscopiques adaptées pour une visualisation du champ opératoire. Ces caméras endoscopiques permettent d'obtenir des images vidéo ou photographiques du champ opératoire, que l'on appelle plus généralement des images endoscopiques.

Sur la figure 1, une seule caméra endoscopique 9 est représentée mais l'on rappelle qu'il pourra en être utilisé une pluralité, notamment lorsque l'on veut obtenir des informations tridimensionnelle (3D) du champ opératoire. En effet les caméras endoscopiques peuvent former des capteurs stéréoscopiques grâce auxquelles des images 3D du champ opératoire peuvent être reconstruites.

La caméra endoscopique 9 est insérée dans la cavité 1 à travers un trocart 10, dédié ou non aux moyens d'imagerie endoscopiques.

La caméra endoscopique 9 peut être manipulée directement par le chirurgien, ou par un assistant dont la tâche est exclusivement de guider les moyens d'imageries endoscopiques pour que le chirurgien puisse rester concentrer sur la tâche chirurgicale qu'il effectue.

Il peut également être utilisé un robot porte endoscope 11 qui est prévu pour opérer un guidage contrôlé de la caméra endoscopique 9 dans la cavité. Le robot porte endoscopie 11 est plus précisément capable de suivre les outils de façon automatisée, par traitement d'image par exemple. Ainsi, il permet au chirurgien d'être réellement exclusivement concentré sur la tâche chirurgicale en cours puisqu'il n'a aucun ordre à donner à l'assistant, le robot porte endoscope étant en effet autonome dans le déplacement de la caméra endoscopique.

Préférentiellement, le robot porte endoscope est de petite taille de sorte qu'il puisse être placé relativement proche du patient, sans gêner le chirurgien. Il pourra par exemple être utilisé le robot porte endoscope VikY commercialisé par la société Endocontrol. Pour une description plus détaillée de ce dispositif, on se réfèrera utilement à la publication de P. Berkelman, P. Cinquin, E. Boidard, J. Troccaz, C. Létoublon, et al. intitulée « Development and testing of a compact endoscope manipulator for minimally invasive surgery » (Journal of Computer Aided Surgery, 2005. 10(1): p.1-13).

Il convient en outre d'utiliser des moyens d'imagerie par ultrasons permettant d'acquérir des images échographiques du champ opératoire, de manière à donner des informations complémentaires au chirurgien pendant son intervention, en vue notamment d'obtenir une visualisation plus précise des organes (2;3;4). Ces moyens d'imagerie par ultrasons permettent en particulier de visualiser les organes (4) qui ne seraient pas du tout visibles ou visibles seulement en partie part le dispositif d'imagerie endoscopique (3;4). En particulier, l'imagerie ultrasons en mode Doppler permet d'identifier facilement les structures vasculaires. Cela peut être tout à fait intéressant par exemple dans la prostatectomie radicale, pour le repérage des « bandelettes vasculo-nerveuses », qu'il faut impérativement préserver, et que le chirurgien a souvent du mal à identifier à partir des seules images endoscopiques.

Une fois encore, on peut utiliser plusieurs dispositifs d'imagerie à ultrasons même si seulement un seul dispositif d'imagerie à ultrasons 12 est représenté à la figure 1.

Ce dispositif d'imagerie à ultrasons 12 peut être placé à l'extérieur du patient. Il pourra également consister en une sonde échographique placée par exemple dans un organe du patient accessible par des voies naturelles (rectum, vessie, urètre, etc.) ou par ponction (vaisseau, espace articulaire, du genou par exemple, etc.). Dans le cas de la prostatectomie radicale, ce dispositif d'imagerie à ultrasons 12 peut ainsi être positionné dans le rectum du patient. Une autre solution est de le placer directement dans la cavité 1 par voie endoscopique.

Tout comme la caméra endoscopique 9, le dispositif d'imagerie à ultrasons 12 peut être porté par un robot permettant de le maintenir en position, et de contrôler son changement de position éventuel.

Le fait d'utiliser un robot porte endoscope de faible encombrement, tel que le système VikY développé par la société Endocontrol cité plus haut, permet de positionner le dispositif à des endroits généralement non accessibles sans pour autant gêner le chirurgien. En outre le volume occupé par les moyens d'imagerie est réduit, ce qui est particulièrement avantageux.

Les différents dispositifs d'imagerie, à savoir la ou les caméras endoscopiques 9, et le ou les dispositifs d'imagerie à ultrasons 12, sont couplés à un dispositif de traitement 13 capable de traiter les différentes informations reçues, c'est à dire à la fois les informations endoscopiques et les informations échographiques.

Ce dispositif de traitement 13 comprend des moyens de calculs permettant de synthétiser les différentes informations reçues pour synthétiser une image enrichie du champ opératoire, cette image étant affichée sur un moyen d'affichage 14 quelconque. L'image est dite enrichie puisqu'elle intègre à la fois des informations endoscopiques et des informations échographiques, de manière à améliorer la visualisation des outils (5;6) et des organes (2;3;4) dans le champ opératoire pour faciliter l'intervention du chirurgien sous endoscopie.

Toutefois, pour pouvoir synthétiser cette image enrichie, les moyens de traitement 13 requièrent des informations supplémentaires permettant une mise en correspondance des informations endoscopiques et des informations échographiques.

Pour cette mise en correspondance, il est proposé d'utiliser des moyens pour créer un référentiel commun aux images endoscopiques et échographiques pour pouvoir mettre en correspondance ces images et donc les informations correspondantes.

Pour ce faire des marqueurs sont utilisés, ces marqueurs étant adaptés pour être visibles à la fois par la caméra endoscopique 9 et par le dispositif d'imagerie à ultrasons 12.

On utilisera plus précisément un minimum de trois marqueurs (15;16;17) de manière à pouvoir opérer une triangulation précise et avoir un positionnement en trois dimensions des informations endoscopiques et échographiques.

Ces marqueurs (15;16;17) étant visibles à la fois sur les images endoscopiques et sur les images échographiques, ils servent de référentiels pour une mise en correspondance des différentes informations. Les moyens de calcul des moyens de traitements 13 se basent sur ces informations communes pour fusionner les informations endoscopiques et échographiques, afin de synthétiser l'image enrichie désirée.

Les marqueurs (15;16;17) sont adaptés pour être insérés dans la cavité 1, au niveau du champ opératoire, de préférence à travers l'un des trocarts (7;8;10). Les marqueurs sont donc adaptés pour être introduits dans la cavité du champ opératoire par voie endoscopique, sans devoir être nécessairement rigidement liés au patient.

Ils sont ensuite positionnés par le chirurgien dans le champ opératoire de manière à être dans le champ d'observation de la caméra endoscopique 9 et du dispositif d'imagerie par ultrasons 12. Ils seront par exemple posés ou fixés sur certains organes dans la zone d'intérêt du champ opératoire. A noter que plus les marqueurs seront proches des organes d'intérêt et plus ils seront facilement visibles sur les images échographiques. Cela permet d'utiliser un dispositif d'imagerie par ultrasons 12 tridimensionnel, dont le champ de « vision » est souvent restreint, ou de minimiser les mouvements nécessaires lorsque le dispositif d'imagerie par ultrasons 12 est bidimensionnel.

Cette solution de mise en correspondance est très simple ; elle nécessite notamment très peu de calculs qui sont rapidement effectués. En outre, l'invention permet à l'opérateur de visualiser en temps quasi-réel, voire en temps réel, la position des outils par rapport aux organes visibles seulement par échographie, puisque les seuls calculs effectués concernent le recalage d'image. Cela est particulièrement avantageux par rapport aux méthodes connues qui permettent un suivi direct de l'outil, en plaçant sur cet outil des dispositifs de suivi spécifiques, tels que des marqueurs spécifiques pouvant être observés par un dispositif d'imagerie par ultrasons.

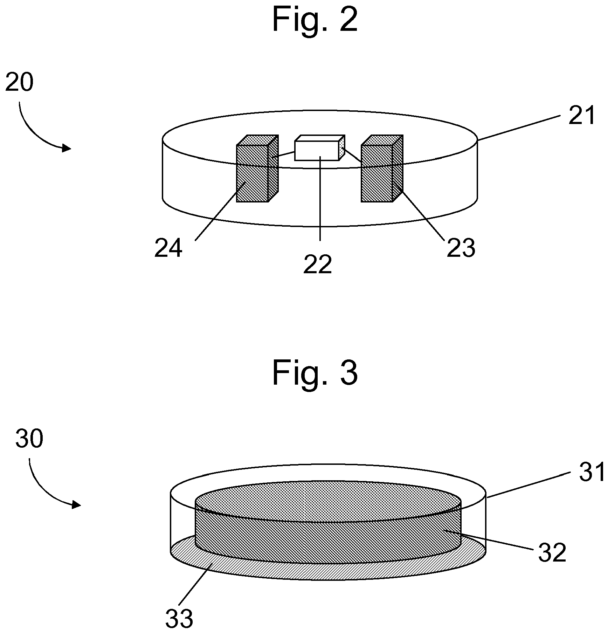

Les marqueurs (15;16;17) peuvent avoir une forme quelconque, l'essentiel étant qu'ils puissent être insérés dans la cavité 1 à travers les trocarts, ou à l'aide d'une aiguille. Ils pourront par exemple avoir la forme de petites pastilles cylindriques comme on peut le voir représenté aux figures 2 et 3. De telles pastilles auront préférentiellement un diamètre et une hauteur inférieurs à 5 mm. Dans le cas de la prostatectomie radicale, on pourra alternativement utiliser des marqueurs composés d'une aiguille dont une extrémité comporte au moins un transducteur capacitif à ultrasons micro-usiné (CMUT, acronyme anglais de « capacitive micromachined ultrasonic transducer »). Le volume très réduit de ces transducteurs permet en effet de les intégrer directement à une aiguille. Chaque aiguille est introduite par voie endoscopique, à travers les tissus qui masquent la prostate par rapport à la caméra endoscopique, jusqu'à ce que le transducteur CMUT soit au contact de la prostate, et de telle sorte qu'une partie suffisante de l'aiguille puisse être vue par la caméra endoscopique.

Il n'est pas nécessaire que les marqueurs (15;16;17) soient connectés entre eux. En effet, il est surtout important que ces marqueurs (15;16;17) soient fixes au cours de chaque acquisition des dispositifs d'imagerie ce qui peut être supposé puisque les temps d'acquisition sont très faibles. Les marqueurs peuvent donc être mobiles les uns par rapport aux autres tant qu'ils peuvent être visualisés par les deux modalités d'imagerie de manière simultanée, ou quasi-simultanée, c'est-à-dire à des intervalles de temps suffisamment brefs pour que leurs mouvements relatifs puissent être négligés. En tout état de cause, cela peut être imposé par une synchronisation temporelle des acquisitions par les dispositifs d'imagerie. L'utilisation de marqueurs distincts, et donc mobiles les uns par rapport aux autres, permettra une introduction plus aisée des marqueurs (15;16;17) dans la cavité 1.

L'intérêt d'utiliser de tels marqueurs servant de référentiel commun aux deux modalités d'imagerie est de pouvoir effectuer des calculs rapides pour estimer les transformations géométriques entre le référentiel des images endoscopiques et le référentiel des images échographiques, ce qui est encore plus avantageux lorsque les informations endoscopiques et échographiques sont obtenues de manière simultanée.

Pour que les temps de calcul de recalage entre chaque acquisition soient encore plus rapides, il est possible de connecter les marqueurs (15;16;17) par des moyens de liaisons plus ou moins souples. Ce mode de réalisation est représenté à la figure 1. Ainsi, les mouvements des marqueurs les uns par rapport aux autres sont réduits de sorte que le recalage est plus simple. Une liaison souple permet en outre de ne pas trop compliquer l'introduction des marqueurs (15;16;17) dans la cavité 1 puisque l'ensemble ainsi constitué peut être déformé relativement simplement.

Pour éviter tout recalage entre les acquisitions, il est également possible de lier rigidement les marqueurs (15;16;17) par des articulations qui permettent l'introduction dans la cavité 1 via les trocarts, tout en assurant une rigidité suffisante après le déploiement.

Comme on l'a dit plus haut, les marqueurs sont adaptés pour être reconnus à la fois par un dispositif d'imagerie photosensible (telle qu'une caméra endoscopique) et par un dispositif d'imagerie par ultrasons (telle qu'une sonde échographique). Plusieurs solutions sont envisageables pour constituer de tels marqueurs.

Une première solution qui est illustrée à la figure 2, est d'utiliser un marqueur 20 ayant une enveloppe 21 enfermant une source d'énergie 22 telle qu'une micro-batterie. Ainsi, la source d'énergie 22 peut être utilisée pour alimenter des dispositifs d'émission de lumière et/ou d'ultrasons. Un tel marqueur est dit marqueur actif.

On pourra en effet prévoir un dispositif 23 émettant un rayonnement qui sera reconnu par la caméra endoscopique ; ce sera typiquement une source lumineuse comme par exemple une diode.

Le marqueur 20 enferme en outre un dispositif 24 d'émission d'ultrasons qui seront reconnus par le dispositif d'imagerie à ultrasons 12, ce dispositif 24 d'émission d'ultrasons étant alimenté par la source d'énergie 22.

Le dispositif 24 d'émission d'ultrasons pourra par exemple être un transducteur piézo-électrique capable d'émettre des ondes ultrasonores dans la gamme de détection du dispositif d'imagerie à ultrasons 12. On pourra aussi utiliser un transducteur capacitif à ultrasons micro-usiné (CMUT, acronyme anglais de « capacitive micromachined ultrasonic transducer ») qui présente l'avantage d'être très petit et de pouvoir donc facilement être incorporé dans une enveloppe 21 de faible volume.

L'enveloppe 21 est fabriquée dans un matériau biocompatible transparent aux ondes ultrasonores et aux ondes lumineuses de sorte que les signaux émis par les dispositifs d'émission lumineuse 23 et ultrasonore 24 puissent être reconnus par les dispositifs d'imageries endoscopiques 9 et échographiques 12.

En outre, le marqueur comprend un système de fixation (non représenté) pour faciliter son accroche dans la cavité 1. On pourra par exemple prévoir un moyen de clipsage ou une colle biologique à la surface de l'enveloppe 21. L'enveloppe 21 peut également avoir une forme particulière facilitant son adhésion aux organes ; elle pourra ainsi avoir une forme de ventouse.

La figure 3 représente un marqueur 30 selon un autre mode de réalisation possible. Dans ce cas, le marqueur n'a pas de source énergétique intégrée, de sorte qu'il est dit marqueur passif.

L'enveloppe 31 présente dans ce cas une cavité qui peut être remplie avec un fluide 32 qui pourra par exemple être un produit de contraste reconnu par le dispositif d'imagerie par ultrasons. Le plus souvent ces produits de contraste sont des émulsions contenant des microbulles d'air, comme par exemple le Levovist™ commercialisé par la société Schering.

Puisque l'enveloppe 31 est transparente aux ultrasons, le produit de contraste se révèlera avec une forte intensité sur les images échographiques, ce qui permettra de positionner avec précision les marqueurs sur l'image échographique.

Une portion de l'enveloppe est recouverte d'une peinture ou d'un matériau rétro-réfléchissant 33 permettant d'augmenter la visibilité et le contraste du marqueur 30 dans les images endoscopiques. Il convient d'utiliser une peinture ou un matériau rétro-réfléchissant 33 biocompatible. La peinture ou le matériau rétro-réfléchissant 33 étant déposée sur l'enveloppe 31, il n'est donc plus forcément nécessaire que l'enveloppe 31 soit formée dans un matériau transparent à la lumière.

Pour améliorer encore plus la visibilité et le contraste du marqueur 30 dans les images endoscopiques, on peut utiliser une peinture ayant des particules fluorophores. En effet, on peut aussi prévoir une source lumineuse d'excitation des fluorophores, cette source lumineuse pouvant être placée sur la caméra endoscopique ou à tout autre endroit. La caméra endoscopique est en outre adaptée pour reconnaître le signal émis par les fluorophores. En outre, l'utilisation de particules fluorophores est particulièrement intéressante si le champ de vision est obstrué par du tissu, au point que le marqueur ne peut être vu par la caméra endoscopique. En effet, en utilisant une source lumineuse émettant un signal lumineux dans la fenêtre de transparence des tissus biologiques (c'est-à-dire avec une longueur d'onde préférentiellement comprise entre 750 nm et 1350 nm), le signal émis par l'excitation des fluorophores pourra être perçu par la caméra endoscopique (adaptée pour ces longueurs d'onde particulières) malgré les tissus gênants.

Il convient de noter que l'utilisation d'une peinture biocompatible pourrait également être envisagée pour le marqueur 20 présenté plus haut et illustré à la figure 2. Cette peinture viendrait en complément ou en remplacement du dispositif d'émission de lumière 23.

Un autre type de marqueur passif est constitué d'un matériel biocompatible destiné à être injecté directement à l'intérieur d'un organe donc lié de manière intrinsèque à cet organe. Ces marqueurs sont dits internes, comparativement aux précédents plus destinés à être déposés à la surface ou au contact d'un organe présent dans le volume dans lequel se trouve le champ opératoire, et qu'on peut par conséquent qualifier de marqueurs externes (par rapport à l'organe). De tels marqueurs internes peuvent par exemple être de nature liquidienne ou sous forme de pâte biocompatible.

De tels marqueurs sont adaptés pour ne pas diffuser dans l'organe après avoir été injectés, de manière à rester dans un volume particulier sous la forme d'un amas par exemple. Ainsi, on peut par exemple utiliser un marqueur liquidien que l'on injecte dans une zone bien choisie d'un organe pour qu'il forme une collection, c'est à dire un amas de ce liquide particulier dans la zone de l'organe. De tels marqueurs peuvent également être formés à partir d'une substance adaptée pour conserver sa forme, et présentant donc une cohésion importante de sorte que le matériau ne se sépare pas. On peut par exemple utiliser un liquide visqueux, c'est à dire présentant une viscosité importante, ou un matériau dont la composition permet d'assurer cette cohésion, tel qu'un gel ou une pâte par exemple.

Naturellement, ces marqueurs doivent être particularisés de manière à être visualisés par les différentes modalités d'acquisition d'imagerie. Ainsi, à titre d'exemple, dans le cas de détecteurs photosensibles, il peut être utilisé de l'eau stérile colorée, par l'utilisation par exemple d'un colorant universel, type bleu de méthylène soluble dans l'eau. Une autre solution consiste à utiliser de l'eau stérile avec incorporation d'un fluorophore biocompatible. Dans ce dernier cas, on choisit des particules fluorophores susceptibles d'être excités par des longueurs d'onde situées dans la « fenêtre de transparence des tissus biologique » (par exemple, entre 700 nm et 800 nm) et de réémettre un signal dans cette même fenêtre de transparence. L'avantage est alors que le « marqueur interne » peut être injecté plus profondément à l'intérieur de l'organe, tout en restant visible par des caméras classiques ou par des caméras sensibles aux infrarouges proches. Ces différents marqueurs liquidiens ou « pâteux » pourront, si nécessaire, être enrichis de produits de contraste ultrasonore biocompatible, par exemple de type microbulles (ce produit de contraste ultrasonore étant de plus en plus utilisé). Ils permettent de sensibiliser la détection échographique. Un produit de contraste peut consister par exemple en un produit totalement opaque aux ultrasons, ce qui permettra aux marqueurs d'être apparents sur l'image échographique.

Ces marqueurs sont ensuite injectés au sein de zones d'organes à l'aide d'un dispositif adapté (aiguille, dispositif de iontophorèse, par exemple) de manière à pouvoir détecter au minimum trois « spots », correspondant à trois collections distinctes dans le cas de marqueurs liquides par exemple.

Un soin particulier sera apporté aux modalités d'injection de manière à maintenir, au moins pendant la durée de la phase de mise en correspondance, un aspect caractéristique de chaque « spot » (par exemple, sous la forme d'une collection) et à une profondeur adaptée aux caractéristiques de détection des systèmes d'imagerie utilisés permettant de les identifier, de manière aisée et univoque, au sein des différentes modalités d'imagerie.

Ces marqueurs passifs internes peuvent ainsi être visualisés, d'une part par le système d'imagerie photosensible du fait des propriétés optiques caractéristiques du matériel injecté, mais aussi par le système ultrasonore suite aux modifications localisées et caractéristiques des impédances acoustiques consécutives à l'injection du marqueur. A titre illustratif et schématique, dans le cas d'injections superficielles au sein de l'organe de bleu de méthylène dissous dans de l'eau stérile, on recherchera respectivement des « tâches » bleues à la surface de l'organe par l'utilisation d'une imagerie photosensible ainsi que des collections hypoéchogènes au niveau de l'imagerie ultrasonore.

Nous allons maintenant décrire une procédure permettant d'obtenir une image enrichie qui aidera le chirurgien pour la manipulation de ses outils chirurgicaux dans le champ opératoire. Par souci de simplicité, cette procédure est présentée dans le cadre de marqueurs « externes » ou de contacts (en opposition avec les marqueurs précédemment identifiés comme « internes »).

On utilise une caméra endoscopique et une sonde échographique. Une sonde échographique 3D sera préférée pour permettre une acquisition du volume d'intérêt à partir d'une unique position de la sonde, sans donc à avoir à la mettre en mouvement. Rappelons que la caméra endoscopique et la sonde échographique pourront être portées par des robots permettant un meilleur maintien en position et offrant la possibilité d'un guidage contrôlé des dispositifs d'imagerie. Dans certains cas, cela permettra également de mobiliser des organes, par exemple en utilisant une sonde échographique endorectale ou endourétrale dans la cadre de la prostatectomie radicale.

Les marqueurs sont ensuite introduits à travers l'un des orifices percutanés et « lâchés » dans le champ opératoire. Les outils chirurgicaux sont ensuite utilisés pour positionner correctement les marqueurs dans le champ opératoire, le plus proche possible de la zone d'intérêt, de manière notamment à que la portion des marqueurs en contact avec les organes permettent aux ultrasons d'être transmis.

A chaque fois qu'une image enrichie doit être synthétisée, il convient d'effectuer les étapes qui suivent.

Tout d'abord, il faut procéder à des acquisitions simultanées d'images endoscopique et échographique, en évitant au maximum des mouvements du champ opératoire.

Ensuite, les informations échographiques acquises sont traitées pour déterminer la position des marqueurs. On peut par exemple déterminer les positions 3D des marqueurs par l'utilisation d'un dispositif 12 d'imagerie ultrasonore 3D, ou tout autre dispositif ou méthode connue de l'homme du métier. L'avantage d'utiliser des marqueurs actifs avec un transducteur (piézo-électrique ou CMUT) est de permettre une segmentation très simple qui ne nécessite pas d'intervention humaine. Au contraire, pour un marqueur passif, il est préférable, au moins au début, d'apporter des informations manuelles. Notons toutefois qu'une segmentation automatique à partir de marqueurs passifs est possible avec une connaissance grossière du positionnement relatif des marqueurs, ce positionnement grossier pouvant être connu grâce aux liaisons plus ou moins souples couplant les marqueurs entre eux. Cette étape permet donc d'obtenir n points tridimensionnels Uᵢ, qui sont les centres des transducteurs ou du produit de contraste.

Notons Ci les n points tridimensionnels correspondant aux centres des dispositifs d'émission de lumière ou des surfaces rétro-réfléchissantes ou peintes sur les marqueurs, et dᵢ les distances entre Uᵢ et Cᵢ. Les valeurs de dᵢ sont connues lors de la fabrication des marqueurs (15;16;17). En observant ces marqueurs par la caméra endoscopique, on obtient n points bidimensionnels Bᵢ, projection des points tridimensionnels Ci dans l'image endoscopique. Une calibration intrinsèque préalable de la caméra endoscopique permet d'en déduire n lignes droites Dᵢ.

La mise en correspondance des images consiste à identifier le vecteur p ayant 6 paramètres (3 rotations et 3 translations) de la matrice reliant les référentiels de la caméra endoscopique et de la sonde échographique. Si on note eᵢ la distance entre le centre Uᵢ et la droite Dᵢ pour chaque marqueur, on remarque que eᵢ est inférieure ou égale à dᵢ lorsque la mise en correspondance est parfaite. Une approximation des paramètres de mise en correspondance peut donc être obtenue par une méthode de moindres carrés non linéaires (par exemple, la méthode de Levenberg-Marquardt), en minimisant ∑eᵢ*eᵢ. Cette approximation sera suffisante lorsque les distances dᵢ sont faibles, typiquement de l'ordre du millimètre (c'est en particulier le cas avec des marqueurs faisant appel à des technologies CMUT, où les transducteurs ultrasonores sont de très faible dimension). Dans le cas où cette approximation ne serait pas suffisante, il suffira d'augmenter le nombre de marqueurs, ou d'utiliser une caméra endoscopique supplémentaire (dans ce dernier cas, il devient en effet possible d'obtenir directement les points Cᵢ).

On notera par ailleurs qu'il peut être intéressant, pour faciliter la mise en correspondance, d'avoir une estimation préalable de l'attitude initiale du référentiel lié à la caméra endoscopique et du référentiel lié à la sonde échographique, pour éviter les minima locaux que l'on rencontre classiquement dans les méthodes de mise en correspondance. Il sera pour cela possible de différencier les marqueurs, par exemple en utilisant des motifs différents (taille, couleur, ...) visibles par la caméra endoscopique, et des caractéristiques ultrasonores différentes pour chaque marqueur (taille, intensité, fréquence, nombre de sources, ...).

Il suffit ensuite de fusionner les informations des deux modalités d'imagerie pour synthétiser l'image enrichie qui sera affichée sur le moniteur de travail du chirurgien. Le chirurgien voit donc non seulement les outils visibles par la caméra endoscopique, mais aussi les organes visibles seulement par les ultrasons.

Dans le cadre de l'utilisation de marqueurs internes, la procédure, adaptée, se déroule selon un processus similaire. A titre d'exemple, nous considérons des marqueurs « internes » contenant des fluorophores. Ces marqueurs sont injectés dans différentes zones d'organes. On détermine ensuite, dans la modalité ultrasonore, les n points tridimensionnels Uᵢ, centres des zones locales ultrasonores à impédance caractéristique des zones d'injection. Rappelons que ces points tridimensionnels Uᵢ sont situés à l'intérieur d'organes.

L'injection des marqueurs « internes » a lieu de façon adaptée, en particulier en termes de profondeur, pour que l'acquisition videosensible du signal caractéristique du marqueur soit effectivement réalisable (à titre d'exemple, utilisation d'un fluorophore biocompatible avec longueur d'onde d'émission dans le proche infrarouge pour des injections profondes). En reprenant les notations précédentes, les centres Ci, points tridimensionnels, apparaissent comme les centres d'émission des zones injectées. Ils sont confondus, dans cette approche avec les points Uᵢ. Les Ci sont observés indirectement par le système d'acquisition, sous la forme de points Bᵢ, situés à la surface de l'organe. La calibration intrinsèque préalable de la caméra endoscopique permet d'en déduire n lignes droites Dᵢ (qui ne sont autres que les droites BᵢCᵢ).

Comme précédemment, la mise en correspondance des images consiste à identifier le vecteur p à 6 paramètres de la matrice reliant les référentiels de la caméra endoscopique et de la sonde échographique par minimisation d'une fonctionnelle quadratique ∑eᵢ*eᵢ où les eᵢ représentent respectivement la distance des points Uᵢ (modalité ultrasonore) aux droites Dᵢ (modalité vidéo).

Le lecteur aura compris que de nombreuses modifications peuvent être apportées sans sortir matériellement des nouveaux enseignements et des avantages décrits ici. Par conséquent, toutes les modifications de ce type sont destinées à être incorporées à l'intérieur de la portée du système d'imagerie présenté.

## Revendications

1. Système d'imagerie pour le suivi d'au moins un outil chirurgical (5;6) dans un champ opératoire à l'intérieur d'un volume (1) dans un corps d'un animal comprenant:
- au moins une caméra endoscopique (9) pour obtenir des informations endoscopiques du champ opératoire,
- au moins un dispositif d'imagerie par ultrasons (12) pour obtenir des informations échographiques du champ opératoire,
- un dispositif de traitement (13) pour le traitement d'informations endoscopiques et échographiques obtenues simultanément par la caméra endoscopique (9) d'une part et le dispositif d'imagerie par ultrasons (12) d'autre part,
**caractérisé en ce qu'**il comprend en outre au moins trois marqueurs (15;16;17) destinés à être positionnés dans le champ opératoire, lesdits marqueurs (15;16;17) étant mobiles par rapport à l'outil (5;6), chaque marqueur étant adapté pour être visualisé à la fois par la caméra endoscopique (9) et par le dispositif d'imagerie par ultrasons (12), lesdits au moins trois marqueurs formant un référentiel commun pour une mise en correspondance par le dispositif de traitement (13) des Informations endoscopiques et des informations échographiques obtenues simultanément.

2. Système selon la revendication 1, **caractérisé en ce qu'**il comprend en outre des moyens de synchronisation temporelle des acquisitions d'information par la caméra endoscopique (9) et par le dispositif d'imagerie par ultrasons (12).

3. Système selon l'une quelconque des revendications 1 ou 2, **caractérisé en ce que** le dispositif de traitement (13) comprend des moyens pour fusionner les informations endoscopiques et les informations échographiques à partir de la mise en correspondance et pour obtenir une représentation du champ opératoire à partir des informations fusionnées.

4. Système selon la revendication 3, **caractérisé en ce qu'**il comprend en outre un dispositif d'affichage (14) de la représentation du champ opératoire à partir des informations fusionnées.

5. Système selon l'une quelconque des revendications 1 à 4, **caractérisé en ce que** les marqueurs (20) sont formés d'une substance biocompatible comprenant un colorant permettant au marqueur (20) d'être reconnu par la caméra endoscopique (9).

6. Système selon l'une quelconque des revendications 1 à 5, **caractérisé en ce que** les marqueurs (20) sont formés d'une substance biocompatible comprenant des particules fluorophores émettant un signal permettant au marqueur (20) d'être reconnu par la caméra endoscopique (9).

7. Système selon l'une quelconque des revendications 1 à 6, **caractérisé en ce que** les marqueurs (20) sont formés d'une substance biocompatible comprenant un produit de contraste reconnu par le dispositif d'imagerie par ultrasons (12).

8. Système selon l'une quelconque des revendications 5 à 7, **caractérisé en ce que** la substance formant les marqueurs (20) présente une cohésion suffisante pour empêcher ladite substance de se séparer.

9. Système selon l'une quelconque des revendications 1 à 4, **caractérisé en ce que** les marqueurs (20) comprennent une enveloppe (21) formée dans un matériau biocompatible transparent aux ultrasons.

10. Système selon la revendication 9, **caractérisé en ce que** les marqueurs (15;16;17) comprennent une source d'énergie (22).

11. Système selon la revendication 10, **caractérisé en ce que** les marqueurs (15;16;17) comprennent un dispositif d'émission d'un signal lumineux (23) reconnu par la caméra endoscopique (9), ledit dispositif d'émission (23) étant alimenté par la source d'énergie (22).

12. Système selon l'une quelconque des revendications 9 ou 11, **caractérisé en ce que** les marqueurs (15;16;17) comprennent un dispositif d'émission d'un signal ultrasons (24) reconnu par le dispositif d'imagerie par ultrasons (12), ledit dispositif d'émission (24) étant alimenté par la source d'énergie (22).

13. Système selon la revendication 12, **caractérisé en ce que** le dispositif d'émission d'un signal ultrasons (24) comprend au moins un transducteur piézo-électrique.

14. Système selon la revendication 12, **caractérisé en ce que** le dispositif d'émission d'un signal ultrasons (24) comprend au moins un transducteur capacitif à ultrasons micro-usiné (CMUT).

15. Système selon l'une quelconque des revendications 9 à 11, **caractérisé en ce que** les marqueurs (15;16;17) présentent une cavité (31) remplie d'un fluide (32) comprenant un produit de contraste reconnu par le dispositif d'imagerie par ultrasons (12).

16. Système selon l'une quelconque des revendications 1 à 15, **caractérisé en ce que** les marqueurs (15;16;17) comprennent une portion (33) recouverte d'un matériau rétro-réfléchissant biocompatible reconnu par la caméra endoscopique (9).

17. Système selon l'une quelconque des revendications 1 à 15, **caractérisé en ce que** les marqueurs (15;16;17) comprennent une portion (33) recouverte d'une peinture biocompatible reconnue par la caméra endoscopique (9).

18. Système selon la revendication 17, **caractérisé en ce que** la peinture comprend des particules fluorophores, lesdites particules fluorophores émettant un signal reconnu par la caméra endoscopique (9).

19. Système selon l'une quelconque des revendications 1 à 18, **caractérisé en ce que** les marqueurs (15;16;17) comprennent des moyens de fixation pour être accrochés à l'intérieur du volume (1) au niveau du champ opératoire.

20. Système selon l'une quelconque des revendications 1 à 19, **caractérisé en ce que** les marqueurs (15;16;17) sont reliés entre eux par une structure pour éviter le mouvement des marqueurs les uns par rapport aux autres.

21. Système selon l'une quelconque des revendications 1 à 20, **caractérisé en ce que** les marqueurs (15;16;17) sont fixes les uns par rapport aux autres.

22. Système selon l'une quelconque des revendications précédentes, **caractérisé en ce que** le dispositif d'imagerie par ultrasons (12) consiste en une sonde destinée à être introduite dans le corps de l'animal.

23. Système selon l'une quelconque des revendications précédentes, **caractérisé en ce qu'**il comprend au moins un robot (11) apte à porter au moins un moyen d'imagerie parmi la caméra endoscopique (9) et le dispositif d'imagerie par ultrasons (12), le robot (11) permettant un déplacement contrôlé dudit moyen d'imagerie.

## Patentansprüche

1. Bildgebungssystem zur Verfolgung mindestens eines chirurgischen Instruments (5; 6) in einem Operationsfeld im Innern eines Volumens (1) in einem Körper eines Tieres, umfassend:
- mindestens eine endoskopische Kamera (9), um endoskopische Informationen des Operationsfelds zu erhalten,
- mindestens eine bildgebende Vorrichtung durch Ultraschall (12), um echographische Informationen des Operationsfelds zu erhalten,
- eine Verarbeitungsvorrichtung (13) für die Verarbeitung endoskopischer und echographischer Informationen, die gleichzeitig von der endoskopischen Kamera (9) zum einen und von der bildgebenden Vorrichtung durch Ultraschall (12) zum anderen erhalten werden,
**dadurch gekennzeichnet, dass** es ferner mindestens drei Marker (15; 16; 17) umfasst, die bestimmt sind, im Operationsfeld positioniert zu sein, wobei die Marker (15; 16; 17) in Bezug zu dem Instrument (5; 6) bewegbar sind, wobei jeder Marker ausgebildet ist, um sowohl von der endoskopischen Kamera (9) als auch von der bildgebenden Vorrichtung durch Ultraschall (12) visualisiert zu sein, wobei die mindestens drei Marker ein gemeinsames Bezugssystem für einen Abgleich durch die Verarbeitungsvorrichtung (13) der gleichzeitig ermittelten endoskopischen Informationen und der echographische Informationen bilden.

2. System nach Anspruch 1, **dadurch gekennzeichnet, dass** es ferner Mittel für die temporäre Synchronisierung der Informationserwerbe durch die endoskopische Kamera (9) und durch die bildgebende Vorrichtung durch Ultraschall (12) umfasst.

3. System nach einem der Ansprüche 1 oder 2, **dadurch gekennzeichnet, dass** die Verarbeitungsvorrichtung (13) Mittel zum Fusionieren der endoskopischen Informationen und der echographischen Informationen auf der Basis des Abgleichs und um eine Darstellung des Operationsfeld auf der Basis der fusionierten Informationen zu erhalten, umfasst.

4. System nach Anspruch 3, **dadurch gekennzeichnet, dass** es ferner eine Anzeigevorrichtung (14) der Darstellung des Operationsfelds auf der Basis der fusionierten Informationen umfasst.

5. System nach einem der Ansprüche 1 bis 4, **dadurch gekennzeichnet, dass** die Marker (20) von einer biologisch kompatiblen Substanz gebildet sind, umfassend einen Farbstoff, der es dem Marker (20) erlaubt, von der endoskopischen Kamera (9) erkannt zu werden.

6. System nach einem der Ansprüche 1 bis 5, **dadurch gekennzeichnet, dass** die Marker (20) von einer biologisch kompatiblen Substanz gebildet sind, umfassend fluorophore Partikel, die ein Signal senden, das es dem Marker (20) erlaubt, von der endoskopischen Kamera (9) erkannt zu werden.

7. System nach einem der Ansprüche 1 bis 6, **dadurch gekennzeichnet, dass** die Marker (20) von einer biologisch kompatiblen Substanz gebildet sind, umfassend ein Kontrastmittel, das von der bildgebenden Vorrichtung durch Ultraschall (12) erkannt wird.

8. System nach einem der Ansprüche 5 bis 7, **dadurch gekennzeichnet, dass** die Substanz, welche die Marker (20) bildet, eine ausreichende Kohäsion aufweist, um zu vermeiden, dass sich die Substanz trennt.

9. System nach einem der Ansprüche 1 bis 4, **dadurch gekennzeichnet, dass** die Marker (20) eine Hülle (21) umfassen, die aus einem biologisch kompatiblen, für Ultraschall transparenten Material gebildet ist.

10. System nach Anspruch 9, **dadurch gekennzeichnet, dass** die Marker (15; 16; 17) eine Energiequelle (22) umfassen.

11. System nach Anspruch 10, **dadurch gekennzeichnet, dass** die Marker (15; 16; 17) eine Sendevorrichtung eines Lichtsignals (23) umfassen, das von der endoskopischen Kamera (9) erkannt wird, wobei die Sendevorrichtung (23) von der Energiequelle (22) versorgt wird.

12. System nach einem der Ansprüche 9 oder 11, **dadurch gekennzeichnet, dass** die Marker (15; 16; 17) eine Sendevorrichtung eines Ultraschallsignals (24) umfassen, das von der bildgebenden Vorrichtung durch Ultraschall (12) erkannt wird, wobei die Sendevorrichtung (24) von der Energiequelle (22) versorgt wird.

13. System nach Anspruch 12, **dadurch gekennzeichnet, dass** die Sendevorrichtung eines Ultraschallsignals (24) mindestens einen piezoelektrischen Wandler umfasst.

14. System nach Anspruch 12, **dadurch gekennzeichnet, dass** die Sendevorrichtung eines Ultraschallsignals (24) mindestens einen kapazitiven mikromechanischen Ultraschallwandler (CMUT) umfasst.

15. System nach einem der Ansprüche 9 bis 11, **dadurch gekennzeichnet, dass** die Marker (15; 16; 17) eine Kavität (31) aufweisen, die mit einem Fluid (32) gefüllt ist, umfassend ein Kontrastmittel, das von der bildgebenden Vorrichtung durch Ultraschall (12) erkannt wird.

16. System nach einem der Ansprüche 1 bis 15, **dadurch gekennzeichnet, dass** die Marker (15; 16; 17) einen Abschnitt (33) umfassen, der mit einem biologisch kompatiblen rückreflektierenden Material bedeckt ist, das von der endoskopischen Kamera (9) erkannt wird.

17. System nach einem der Ansprüche 1 bis 15, **dadurch gekennzeichnet, dass** die Marker (15; 16; 17) einen Abschnitt (33) umfassen, der mit einer biologisch kompatiblen Farbe bedeckt ist, die von der endoskopischen Kamera (9) erkannt wird.

18. System nach Anspruch 17, **dadurch gekennzeichnet, dass** die Farbe fluorophore Partikel umfasst, wobei die fluorophoren Partikel ein Signal senden, das von der endoskopischen Kamera (9) erkannt wird.

19. System nach einem der Ansprüche 1 bis 18, **dadurch gekennzeichnet, dass** die Marker (15; 16; 17) Befestigungsmittel umfassen, um im Innern des Volumen (1) im Bereich des Operationsfelds befestigt zu sein.

20. System nach einem der Ansprüche 1 bis 19, **dadurch gekennzeichnet, dass** die Marker (15; 16; 17) durch eine Struktur miteinander verbunden sind, um die Bewegung der Marker im Verhältnis zueinander zu vermeiden.

21. System nach einem der Ansprüche 1 bis 20, **dadurch gekennzeichnet, dass** die Marker (15; 16; 17) im Verhältnis zueinander befestigt sind.

22. System nach einem der vorangehenden Ansprüche, **dadurch gekennzeichnet, dass** die bildgebende Vorrichtung durch Ultraschall (12) aus einer Sonde besteht, die bestimmt ist, in den Körper des Tieres eingeführt zu sein.

23. System nach einem der vorangehenden Ansprüche, **dadurch gekennzeichnet, dass** es mindestens einen Roboter (11) umfasst, der imstande ist, mindestens ein bildgebendes Mittel, die endoskopische Kamera (9) oder die bildgebende Vorrichtung durch Ultraschall (12), zu tragen, wobei der Roboter (11) eine kontrollierte Verlagerung des bildgebenden Mittels erlaubt.

## Claims

1. An imaging system to monitor at least a surgical instrument (5; 6) in an operative site inside a volume (1) in the body of an animal, comprising:
- at least one endoscopic camera (9) to obtain endoscopic data on the operative site,
- at least one ultrasound imaging device (12) to obtain ultrasound data on the operative site,
- a processing device (13) for processing endoscopic and ultrasound data obtained simultaneously by the endoscopic camera (9) on one hand and the ultrasound imaging device (12) on the other hand,
**characterized in that** it further comprises at least three markers (15; 16; 17) intended to be positioned in the operative site, said markers (15; 16; 17) being mobile relative to the instrument (5; 6), each marker being adapted to be detected both by the endoscopic camera (9) and by the ultrasound imaging device (12), said at least three markers (15; 16; 17) forming a common reference system for a cross-mapping of the endoscopic and ultrasound data obtained simultaneously.

2. The system of claim 1, **characterized in that** it further comprises means for time synchronization of the data acquisitions by the endoscopic camera (9) and by the ultrasound imaging device (12).

3. The system of either of claims 1 or 2, **characterized in that** the processing device (13) comprises means to merge the endoscopic data and ultrasound data from the cross-mapping and to obtain a representation of the operative site from the merged data.

4. The system of claim 3, **characterized in that** it further comprises a display device (14) to display the representation of the operative site from the merged data.

5. The system of any of claims 1 to 4, **characterized in that** the markers (20) are formed of a biocompatible substance containing a dye allowing the marker (20) to be recognized by the endoscopic camera (9).

6. The system of any of claims 1 to 5, **characterized in that** the markers (20) are formed of a biocompatible substance containing fluorophore particles emitting a signal allowing the marker (20) to be recognized by the endoscopic camera (9).

7. The system of any of claims 1 to 6, **characterized in that** the markers (20) are formed of a biocompatible substance containing a contrast product recognized by the ultrasound imaging device (12).

8. The system of any of claims 5 to 7, **characterized in that** the substance forming the markers (20) has sufficient cohesion to prevent said substance from separating.

9. The system of any of claims 1 to 4, **characterized in that** the markers (20) comprise an envelope (21) formed in a biocompatible material transparent to ultrasound.

10. The system of claim 9, **characterized in that** the markers (15; 16; 17) comprise an energy source (22).

11. The system of claim 10, **characterized in that** the markers (15; 16; 17) comprise a device (23) emitting a light-signal recognized by the endoscopic camera (9), said emitting device (23) being powered by the energy source (22).

12. The system of any of claims 9 or 11, **characterized in that** the markers (15; 16; 17) comprise a device (24) emitting an ultrasound signal recognized by the ultrasound imaging device (12), said emitting device (24) being powered by the energy source (22).

13. The system of claim 12, **characterized in that** the ultrasound signal emitting device (24) comprises at least one piezoelectric transducer.

14. The system of claim 12, **characterized in that** the ultrasound signal emitting device (24) comprises at least one capacitive micro-machined ultrasonic transducer (CMUT).

15. The system of any of claims 9 to 11, **characterized in that** the markers (15; 16; 17) have a cavity (31) filled with a fluid (32) containing a contrast product recognized by the ultrasound imaging device (12).

16. The system of any of claims 1 to 15, **characterized in that** the markers (15; 16; 17) comprise a portion (33) coated with a biocompatible retroreflective material recognized by the endoscopic camera (9).

17. The system of any of claims 1 to 15, **characterized in that** the markers (15; 16; 17) comprise a portion (33) coated with biocompatible paint recognized by the endoscopic camera (9).

18. The system of claim 17, **characterized in that** the paint contains fluorophore particles, said fluorophore particles emitting a signal recognized by the endoscopic camera (9).

19. The system of any of claims 1 to 18, **characterized in that** the markers (15; 16; 17) comprise attachment means for adhering inside the volume (1) of the operative site.

20. The system of any of claims 1 to 19, **characterized in that** the markers (15; 16; 17) are connected together by a structure to avoid movement of the markers relative to one another.

21. The system of any of claims 1 to 10, **characterized in that** the markers (15; 16; 17) are fixed relative to one another.

22. The system of any of the preceding claims, **characterized in that** the ultrasound imaging device (12) consists of a probe intended to be inserted inside the body of the animal.

23. The system of any of the preceding claims, **characterized in that** it comprises a robotic manipulator (11) capable of carrying at least one imaging means from among the endoscopic camera (9) and the ultrasound imaging device (12), the manipulator (11) permitting controlled movement of said imaging means.
